# EUROPEAN PATENT APPLICATION

(11) **EP 0 979 830 A1**
(43) Date of publication of application: **16.02.2000**
(21) Application number: 98202704.7
(22) Date of filing: 12.08.1998
(51) Int. Cl.: C07K 14/415, C12N 9/24, A23L 1/105, C07K 1/36, C07K 16/16, G01N 33/53

(54) **A novel class of xylanase inhibitors**

(71) Applicant: NEDERLANDSE ORGANISATIE VOOR TOEGEPAST-NATUURWETENSCHAPPELIJK ONDERZOEK TNO, 2628 VK Delft (NL)
(72) Inventor: Hessing, Martin, 3994 ED Houten (NL); Happe, Randolph Peter, 1508 WN Zaandam (NL)
(74) Representative: de Bruijn, Leendert C.

(57) **Abstract**

The invention relates to a novel class of xylanase-inhibiting proteins, capable of forming a stable complex with endo-xylanases, thereby inactivating the latter. The inhibitors can be applied as stabilising agents to xylan-degrading enzymes used for industrial processes, e.g for food, feed and non-food applications as paper and pulp technology. Furthermore, the invention relates to strain improvement of industrial xylanase-producing organisms as well as to the selection of cereals, in particular wheat, in which xylanase-inhibiting proteins are absent. Finally the invention relates to quantification and control of xylanase inhibitors for assuring effective and controlled dosing of xylanases applied for various industrial processes.

## Description

### FIELD OF THE INVENTION

The invention relates to the complex formation, leading to inhibition of the xylan-degrading activity, of xylan-degrading enzymes with novel xylanase-inhibiting proteins ("xylanase inhibitors"), the latter being endogenously present in cereals, in particular in wheat bran or fractions thereof. The invention also relates to a method for purifying these proteinaceous inhibitors to homogeneity, as well as to the use of these inhibitors as stabilisers for xylan-degrading enzymes, applied in food, feed and non-food as paper, and pulp technology. Furthermore the invention relates to methods for strain improvement of industrial xylanase-producing organisms. The invention also relates to methods for the selection of cereals, e.g. wheat, in which xylanase-inhibiting proteins do not occur. Finally the invention relates to quantification and control of xylanase inhibitors for assuring effective and controlled dosing of xylanases applied for various industrial processes.

### BACKGROUND OF THE INVENTION

It has been known for a long time that non-starch polysaccharides (NSPs) from wheat flour have an impact on the bread-making process (Pence et al 1950; Udy 1956, 1957; Jelaca and Hlynka 1971). It is generally accepted that NSPs bind a significant amount of water and influence the visco-elasticity of doughs (Kulp 1968a; Jelaca and Hlynka 1971). There are, however, discrepancies in literature regarding their impact on bread loaf volume, crumb characteristic, crust colour and staling of bread (D'Appolonia et al 1970; D'Appolonia 1971, 1973, 1980; Casier et al 1973, 1979; Kim and D'Appolonia 1977a,b; Meuser and Suckow 1986). NSP-hydrolysing enzymes are increasingly employed in the bread making industry (MeClearly 1986; Ter Haseborg and Himmelstein 1988; Maat et al 1992). Such enzymes, when added in optimal quantities, clearly improve the machinability of doughs and the volume and appearance of breads (Maat et al 1992; Gruppen et al 1992).

NSPs also influence the digestibility of feeds rich in cereals and legumes (in particular rye or wheat). Production of sticky dropping and poor growth and feed conversion, especially in younger broilers are often related with these compounds (Moran et al 1969; Campbell et al 1983; Fengler et al 1988; Pettersson et al 1988). Addition of enzyme preparations of microbial origin capable of degrading these NSPs has been shown to considerably improve the growth and feed conversion of chicks fed rye-based diets (Fengler et al 1988; Grootwassink et al 1989). The effect is mainly attributable to the reduction of intestinal viscosity.

The use of NSP-attacking enzymes in the pulp and paper industry has become more and more important (Wong et al 1988) due to their bleach boosting properties helping to replace the use of damaging bleaching agents (Farrell et al 1992; Nissen et al 1992; Wong and Saddler 1992). Other applications of these NSP-hydrolysing enzymes are clarifying juices, beers and wines, extracting coffee, plant oils and starch, and producing food thickeners (Wong and Saddler 1992).

The described applications explain why the use of xylanases for industrial processes are of economical value. Understandingly, endo-xylanases, and NSP-hydrolysing enzymes in general, have received a great deal of attention.

Measurements of the activity of xylanases in feed are usually troublesome, as is monitoring the effect of xylanases applied to feed on animal performance, not only due to the interference of endogenously present polysaccharides. There are various indications that factors present in wheat are potential inhibitors of endo-xylanases, i.e. often no or hardly any activity can be traced back when endo-xylanase is mixed with wheat. A possible explanation could be that xylanases are inactivated due to binding to the factors, present in wheat and extracts thereof (Debyser et al 1997). On basis of the thermolability, the authors of the latter publication suggested that the factors might be proteins.

### DESCRIPTION OF THE INVENTION

The invention relates to proteins inhibiting xylan-degrading enzymes by complex formation. These novel xylanase-inhibiting proteins are obtainable by extraction of cereals, such as wheat, corn, barley, triticale, rice, rye, oat and legumes such as soybeans. They are especially obtainable from wheat bran. These proteins have apparent molecular weights between 20 and 40 kDa, especially between 25 and 35 kDa, in particular between 28 and 32 kDa, and a pI above 8, especially above 9. The proteins especially inhibit endo-xylanases (EC 3.2.1.8).

The proteins are furthermore characterised by having an N-terminal amino acid sequence showing at least 80%, preferably at least 90%, especially at least 96% amino acid identity with the sequence shown in SEQ ID No. 1.

The invention also relates to nucleotide sequences encoding these proteins or parts thereof, either as such, or integrated in a suitable vector or in a recombinant (micro)organism capable of expressing the protein or part thereof.

The invention further relates to the production of these xylanase inhibitors and of their use as stabilisers of xylan-degrading enzymes used for industrial processes, e.g. for food, feed and non-food applications as paper and pulp technology. The invention also pertains to the use of these inhibitors as biocides, e.g. against microorganisms or parasites which depend on xylan degradation for their survival.

Furthermore, the invention relates to strain improvement of industrial xylanase-producing organisms in order to obtain xylanases which are not inhibited by xylanase inhibitors. This improvement can be effected by selecting xylanase-producing strains in which the xylanase in not inhibited by the xylanase-inhibiting proteins of the invention.

Moreover, the invention relates to the selection of cereals, in particular wheat, in which xylanase-inhibiting proteins are absent, as well. The production of non xylanase-inhibiting strains or variants can also performed by defunctionalising the gene encoding the inhibiting protein using conventional recombinant techniques,. to produce transgenic plants.

Also, the invention relates to methods for the detection, quantification and control of xylanase inhibitors, used to predict the resulting activity of xylanases applied for industrial processes, and used for optimising the dosages of xylanase applied for industrial processes. These methods may involve the use of the present proteins or parts thereof as antigens, or antibodies raised against these, in immuno-assays, or of (oligo)nucleotides encoding (parts) of these proteins in hybridisation or other DNA tests.

### EXAMPLES

### Example 1: Isolation and purification of xylanase inhibitors from wheat

Wheat Xylanase Inhibitors were purified from wheat bran, the latter of which was obtained by grinding of 1 kg of wheat. Xylanase-inhibiting activity was determined by monitoring the inhibiting potential of fractions on endo-xylanase activity in the Xylanase AX assay. This assay was carried out as follows: A tablet of Xylazyme AX (containing 60 mg Azurine-crosslinked wheat arabinoxylan) was added to a xylanase solution in 0.5 ml 50 mM acetic acid/NaOH pH 4.7. The reaction mixture was incubated at 40 °C for 10 mm, and subsequently the reaction was stopped by addition of 10 ml 2% TRIS-base (pH 10.8). After filtration, the amount of chromophore released by xylanase activity was determined by measuring the absorbance at 590 nm. A similar incubation mixture without xylanase was used as reference.
All purification steps were performed at room temperature, using BioPilot and FPLC equipment (Pharmacia, Sweden). Wheat bran (200 g; particle size < 0.5 mm) was extracted with 1 l of 0.1 M sodium acetate pH 5.0 by stirring for 1 hour at 4 °C. The suspension was centrifuged at 25,000 g for 20 min, and 400 ml supernatant was obtained. Remnants of bran were removed by filtration (filter paper). Subsequently, ammonium sulphate was added to the bran-free extract to a final concentration of 70%. The precipitate containing the xylanase inhibitors was collected by centrifugation at 25,000 g for 20 min. The pellet was resuspended in 250 ml 20 mM MOPS buffer pH 7.0 and desalted in this buffer using a BPG 100 column packed with 1 l P6 desalting gel. The desalted sample was applied to a BioPilot 35/100 column packed with 100 ml Source 15S, and equilibrated with 300 ml 20 mM MOPS pH 7.0. The column was eluted at a flow rate of 15 ml/min using a linear gradient of 0 - 0.2 M NaCl in 20 mM MOPS buffer pH 7.0 (2 l). Fractions with a volume of 15 ml were collected. Using the Xylanase AX assay, the elution profile from the Source S column invariably showed two protein peaks, displaying inhibiting capability. The two protein peaks were pooled and further purified separately. The pools were desalted in 20 mM MOPS pH 7.0 using a 180 ml P6 column and applied to a HR 5/20 column packed with 4 ml Source 15S for concentration. The column was eluted with a linear gradient of 0 - 0.5 M NaCl in 20 mM MOPS pH 7.0 (8 ml). Subsequently, the samples were applied to a XK26/60 column packed with 320 ml Superdex75 prep grade, equilibrated in 20 mM MOPS pH 7.0, 0.1 M NaCl. The column was eluted with the mentioned buffer at a flow rate of 0.5 ml/min and fractions of 3 ml were collected. The xylanase inhibiting fractions of the highest purity were selected on basis of their performance in the xylanase inhibitor assay and their apparent purity based on SDS-PAGE gel electrophoresis.

This procedure yielded two xylanase-inhibiting protein fractions, referred to as Wheat Xylanase Inhibitor I (WXI-1) and Wheat Xylanase Inhibitor 2 (WXI-2), both displaying a clear single protein band on SDS-polyacrylamide gels. WXI-1 and WXI-2 were found to have MW's of 31 and 29 kDa, respectively, and both inhibitors have pI's higher than 9.
The N-terminal sequences of the inhibitors WXI-1 and WXI-2 is given in SEQ ID NO.1 (AGGKTGQVTVFWGRNKAEGSLREAxDSGMYTMVTMSFLDVFGANGKYHLD). The N-terminal protein sequence data show 90 % similarity and 67 % identity with the internal 29-70 amino-acid sequence of chitinase from *Oryza sativa* (Rice), and also show high homology to other plant chitinases, e.g. from rice, maize and tobacco plants. As the N-terminal sequence of WXI-1 and WXI-2 are identical, these proteins may be the products of the same gene, but have been differently processed.

### Example 2: Complex formation of wheat xylanase inhibiting proteins with endo-xylanases, leading to the inactivation of the latter

Wheat xylanase-inhibiting proteins were able to (almost) completely inactivate certain endo-xylanases, after a short incubation (5 min) at ambient temperatures. Studies were performed by means of gel-filtration chromatography: one of the inhibitors, an endo-xylanase and an incubation mixture of both (after incubation) were applied on and eluted over a Superdex column, in 20 mM Bis-TRIS-HCl pH 6.6 containing 0.1 M NaCl. The elution pattern of the enzyme-inhibitor mixture displayed a new peak at a higher molecular weight position than either the enzyme or the inhibitor. This new peak was identified as the enzyme-inhibitor complex. The appearance of this peak went in concert with the disappearance or decrease of the peaks of the enzyme and the inhibitor. Thus, the complexes remain associated during elution of the column in 0.1 M of salt. In contrast on SDS-PA gels, both under reducing and non-reducing conditions, dissociation of the complexes was detected: protein bands were present at the original positions of the enzyme and of the inhibitor exclusively. Iso-electric focussing displayed a divalent result: WXI-2 and endo-xylanase 1 showed only bands at the original positions of both inhibitor and enzyme (pI's at about 3 and 9). In contrast, on the IEF of WXI-2 with endo-xylanase 1 bands were observed at an intermediate position only, and it was therefore concluded that the complex was not dissociated due to subjection to IEF. The different behaviour of the enzymes is not understood at present.
Inhibiting activity of both WXI-I and WXI-2 was lost upon cooking. This thermo-lability is, as mentioned earlier, additional evidence for the inhibitors being proteins. Addition of EDTA to the incubation mixture, did not result in a decrease of inhibiting activity. The latter indicates that the inhibiting effect is not caused by metals, which are well-known inhibitors of xylan-degrading enzymes. It is concluded that xylanase-inhibiting proteins inhibit particular endo-xylanases by the formation of a complex having a non-covalent character, being stable at pH 6 in 0.1 M NaCl. On basis of N-terminal sequencing, the complex was found to have a 1:1 enzyme to inhibitor ratio.

### Example 3: Effect of xylanase-inhibiting proteins on various commercially available enzymes: screening for enzymes not inhibited by the novel xylanase inhibitor

WXI-1 and WXI-2 were incubated with feed- and bakery xylanase preparations from various sources, e.g. fro*m Trichoderma viride, Trichoderma longibrachiatum, Aspergillus niger, Humicola insolens*, and the effect of the xylanase-inhibiting proteins was evaluated using the Xylazyme AX assay carried out at pH 4.7. Except for one, the inhibitors either both did or did not inhibit a particular enzyme. This method can be used for the improvement of industrial xylanase-inhibiting xylanase producing organisms by selecting xylanases which can not be inhibited by the xylanase inhibitors.

### Example 4: Antibodies raised against wheat xylanase inhibitors

Two New Zealand White rabbits (3 kg) were immunised with 100 µg of protein in 2 ml of antigen solution (FCA). Prior to immunisation, presera were taken. 14 days after immunisation, the titre of the sera was determined with a direct ELISA assay, using WXI-1 antigen as coat. Three weeks after the immunisation, the first booster, containing 100 µg of protein in 2 ml of antigen solution (FIA), was injected into the rabbits. After 10 days the titre of the sera were determined as described above. Three weeks after the first booster, an identical second booster was injected, and after another 11 days the sera were collected. Two lines of polyclonal antibodies against WXI-1 were obtained (rabbit no. 6169 and 6170). These sera were tested by immunoblotting for their reactivity against WXI-1 and WXI-2, which were both recognised. Also, the obtained antibodies can be used to identify and quantify xylanase-inhibiting proteins in other cereals, legumes and from other plant, animal or microbial origin.

### REFERENCES

Casier, J.P.J., De Paepe, G.M.J. and Brummer, J. 1973, Getreide Mehl Brot 27: 36-44.
Casier, J.P.J., De Paepe, G.M.J., Willems, H.E.J., Goffings, G.J.G., Hermans, J.L. and Noppen, H.E. 1979, Trop. Coods Chem. Nutr. 1: 279-340.
Campbell, G.L., Classen, H.L. and Goldsmith, K.A. 1983, Poult. Sci. 62: 2218-2223.
D'Appolonia, B.L., Gilles, K.A. and Medealf, D.G. 1970, Cereal Chem. 47: 194-204.
D'Appolonia, B.L. 1971, A review. Baker's Dig. 45: 20-23, 63.
D'Appolonia, B.L. 1973, Cereal Chem. 50: 27-36.
D'Appolonia, B.L. 1980, Journal of Texture Studies 10: 201-216.
Debyser, W., Derdelinckx, G. and Delcour, J.A. 1997, J. Am. Soc. Brew. Chem. 55: 153-156
Farell, R.L. and Skerker, P.S. 1992, Chlorine-free bleaching wih Cartazyme^{™} HS treatment.
Fengler, A.I. and Marquardt, R.R. 1988, Cereal Chem. 65: 298-302. Pages 315-324 in Xylans and Xylanases, J. Visser et al, eds. Elsevier: Amsterdam.
Grootwassink, J.W.D., Campbell, L.D. and Claessen, H.L. 1989, Poult. Sci. 70: 1571-1577.
Jelaca, S.L. and Hlyncka, I. 1971, Cereal Chem. 48: 211-222.
Gruppen, H., Hamer, R.J. and Voragen, A.G.J. 1992, J. Cereal Sci. 16: 53-67.
Kim, S.K. and D'Applonia, B.L. 1977a, Cereal Chem. 49: 225-229.
Kim, S.K. and D'Applonia, B.L. 1977b, Cereal Chem. 54: 150-160.
Kulp, K. 1968a, Cereal Sci. Today 13: 414-417, 426.
Maat, J., Roza, M., Verbakel, J., Stam, H., santos da Silva, M.J., Bosse, M.,
Egmond, M.R. and Hangemans, M.L.D. 1992, Xylanases and their application in bakery, pages 349-360 in: Xylans and Xylanases, J. Visser et al, eds. Elsevier: Amsterdam.
McClearly, B.V. 1986, Int. J. Biol. Macromol. 8: 349-354.
Meuser, F. And Suckow, P. 1986, Non-starch polysaccharides, pages 42-61 in: Chemistry and Physics of Baking. J.M.V. Blanshard, P.J. Frazier and T. Gaillard, eds. r. Chem. Soc.: London.
Moran, E.T., Lall, S.P. and Summers, J.D. 1969, Poult. Sci 48: 939-949.
Nissen, A.M., Anker, L., Munk, N. and Lange, N.K. 1992, Xylanases for the pulp and paper industry, pages 325-337 in: Xylans and Xylanases, J. Visser et al, eds. Elsevier: Amsterdam.
Pence, I.A. and MacDougall, M. 1958, J. Inst. Brew. 64: 489-500.
Petterson, D. and Aman, P. 1988, Anim. Feed Sci. Technol. 20: 313-324.
Ter Haseborg, E. and Himmelstein, A. 1988, Cereal-Foods-World.St. Paul, Minn.: American Association of Cereal Chemists. May 1988. v. 38 (5) p. 419, 420-421.
Udy, D.C. 1956, Cereal Chem. 33: 67-74.
Udy, D.C. 1957, Cereal Chem. 34: 34-46.
Wong, K.K.Y., Tan, L.U.L. and Saddler, J.N. 1988, Microbiol. Rev. 52: 305-317.
Wong, K.K.Y. and Saddler, J.N. 1992, Trichoderma xylanases, their properties and application, pages 171-186 in: Xylans and Xylanases, J. Visser et al, eds. Elsevier: Amsterdam.

SEQUENCE LISTING

(1) GENERAL INFORMATION

(i) APPLICANT
(A) NAME: Nederlandse Organisatie voor Toegepast
Natuurwetenschappelijk Onderzoek TNO
(B) ADRES: Schoemakerstraat 97
(C) CITY: Delft
(D) COUNTRY: The Netherlands
(E) POSTAL CODE: 2628 VK
(F) TELEPHONE: +31-15-2694961

(ii) TITLE OF THE INVENTION:

A NOVEL CLASS OF XYLANASE INHIBITORS

(iii) NUMBER OF SEQUENCES: 1

(2) INFORMATION ON SEQ ID NO: 1

(i) SEQUENCE CHARACTERISTICS
(A) LENGTH: 50 amino acids
(B) TYPE: amino acid
(C) TOPOLOGY: linear

(ix) FEATURE:
(A) AMINO ACID: Xaa
(B) POSITION: 25
(C) KEY: unknown, possibly cysteine

Ala Gly Gly Lys Thr Gly Gln Val Thr Val Phe Trp Gly Arg Asn
1               5                   10                  15

Lys Ala Glu Gly Ser Leu Arg Glu Ala Xaa Asp Ser Gly Met Tyr
                20                  25                  30

Thr Met Val Thr Met Ser Phe Leu Asp Val Phe Gly Ala Asn Gly
                35                  40                  45

Lys Tyr His Leu Asp
                50

## Claims

1. A xylanase-inhibiting protein, characterised by having an apparent molecular weight of between 20 and 40 kDa, and an N-terminal amino acid sequence showing at least 80% identity with the amino acid sequence given in SEQ ID NO. 1.

2. A xylanase-inhibiting protein according to claim 1, having an apparent molecular weight of between 25 and 35 kDa, especially between 28 and 32 kDa, and/or a pI above pH 9, and/or having an N-terminal amino acid sequence showing at least 90% identity with the amino acid sequence given in SEQ ID NO. 1.

3. A process for obtaining xylanase-inhibiting proteins from cereals, e.g. wheat, comprising extracting the cereals, in particular wheat bran, using an aqueous solvent and selectively precipitating proteinaceous material from the extract obtained, followed by further fractioning the precipitate, and selecting the proteins having an apparent molecular between 25 and 35 kDa, on the basis of SDS-PA gel electro-phoresis, and a pI above 9.

4. Use of a xylanase-inhibiting protein according to claim 1 or 2 as a stabiliser of xylan-degrading enzymes used for the treatment of cereals, such as for animal feedstuffs, or as a stabiliser of xylan-degrading enzymes used in the brewing process, as a bread improver, as a natural paper bleaching agent, for the production of xylose.

5. Use of a xylanase-inhibiting protein according to claim 1 or 2 as a biocide.

6. A method for the improvement of industrial xylanases by selecting xylanases, in particular endo-xylanases, that are not inhibited by a xylanase-inhibiting protein according to claim 1 or 2.

7. A method for the improvement of industrial xylanase-producing organisms by selecting or producing organisms which produce xylanases, in particular endo-xylanases, that are not inhibited by a xylanase-inhibiting protein according to claim 1 or 2, and do not produce xylanases that are inhibited by a xylanase-inhibiting protein according to claim 1 or 2.

8. A method for the improvement of industrial cereals by selecting or producing strains which do not express xylanase-inhibiting proteins.

9. A method for assaying the resulting activity of a xylan-degrading enzyme for the treatment cereals, e.g. wheat, by determining the binding of said xylan-degrading enzyme with a xylanase-inhibiting protein according to claim 1 or 2.

10. An antibody raised against a xylanase-inhibiting protein according to claim 1 or 2.

11. A method for assaying the xylanase-inhibiting activity of proteinaceous material, using an antibody according to claim 9.

12. A nucleotide sequence encoding a xylanase-inhibiting protein according to claim 1 or 2.
